Europäisches Patentamt

European Patent Office

Office européen des brevets

(11) Publication number: **0 503 646 A1**

## EUROPEAN PATENT APPLICATION

(21) Application number: **92104318.8**

(22) Date of filing: **12.03.92**

(51) Int. Cl.⁵: **C07K 15/28**, C07K 3/20,
C12P 21/08, C12N 5/18,
A61K 39/395, A61K 49/00,
G01N 33/53

(30) Priority: **12.03.91 US 667975**

(43) Date of publication of application:
**16.09.92 Bulletin 92/38**

(84) Designated Contracting States:
**AT BE CH DE DK ES FR GB GR IT LI LU MC
NL PT SE**

(71) Applicant: **BIOGEN, INC.**
**14 Cambridge Center**
**Cambridge Massachusetts 02142(US)**

(72) Inventor: **Sato, Vicki L.**

**43 Larch Road**
**Cambridge, Massachusetts 02139(US)**
Inventor: **Chisholm, Patricia L.**
**217 Liberty Street**
**Ouincy, Massachusetts 02169(US)**
Inventor: **Wallner, Barbara P.**
**7 Centre Street**
**Cambridge, Massachusetts 02139(US)**

(74) Representative: **Vossius & Partner**
**Siebertstrasse 4 P.O. Box 86 07 67**
**W-8000 München 86(DE)**

(54) **Monoclonal antibodies recognizing lymphocyte function associated antigen-3.**

(57) Monoclonal antibodies recognizing LFA-3 and capable of blocking adhesion of LFA-3-expressing cells to lymphocytes are described. Such antibodies are useful, e.g., in the treatment of disorders characterized by LFA-3-mediated adhesion of lymphocytes and in diagnostic methods and kits.

Fig. 3

This invention relates to monoclonal antibodies (MAbs) directed to various epitopes of Lymphocyte Function Associated Antigen-3 ("LFA-3"). These novel MAbs are useful in research, in diagnostic and therapeutic compositions, and in purification and other methods of this invention.

T-lymphocytes play a major role in the immune response by interacting with target and antigen-presenting cells (also called effector cells). For example, T-lymphocyte-mediated killing of target cells is a multi-step process involving, initially, adhesion of cytolytic T-lymphocytes (the effector cells) to target cells. Also, helper T-lymphocytes help initiate the immune response by adhesion to antigen-presenting cells.

These interactions of T-lymphocytes with target and antigen-presenting cells are highly specific and depend on the recognition of an antigen on the surface of a target or antigen-presenting cell by one of the many specific antigen receptors on the surface of T-lymphocytes.

The receptor-antigen interaction of T-lymphocytes and other cells is also facilitated by various T-lymphocyte surface proteins, e.g., the antigen-receptor complex CD3 and accessory molecules such as CD4, LFA-1, CD8, and CD2. It is also dependent on accessory molecules such as LFA-3, ICAM-1 and MHC that are expressed on the surface of the target or antigen-presenting cells.

It has recently been demonstrated that accessory molecules on T-lymphocytes and on target or antigen-presenting cells interact with each other to mediate intercellular adhesion. Accordingly, these accessory molecules are thought to enhance the efficiency of lymphocyte/antigen-presenting cell and lymphocyte/target cell interactions and to be important in leukocyte-endothelial cell interaction and lymphocyte recirculation.

For example, recent studies have suggested that there is a specific interaction between CD2 (a T-lymphocyte accessory molecule) and LFA-3 (a target cell accessory molecule) which mediates T-lymphocyte adhesion to the target cell. This cell-cell adhesion is essential to the initiation of the T-lymphocyte functional responses (Dustin et al., J. Exp. Med., 165, pp. 677-692 (1987); Springer et al., Ann. Rev. Immunol., 5, pp. 223-252 (1987)). The LFA-3/CD2 interaction plays a role in mediating T-lymphocyte interactions with thymic epithelial cells, in antigen-independent and dependent conjugate formation and in T-lymphocyte rosetting with erythrocytes (see, e.g., Seed et al., Proc. Natl. Acad. Sci. USA, 84, pp. 3365-3369 (1987)).

LFA-3, which is found on the surface of a wide variety of cells, including human erythrocytes, has become the subject of a considerable amount of study to further elucidate its role in various T-lymphocyte interactions (see, e.g., Krensky et al., J. Immunol., 131(2), pp. 611-616 (1983); Shaw et al., Nature, 323, pp. 262-264 (1986)). Two natural forms of LFA-3 have been identified. One form of LFA-3 ("transmembrane LFA-3") is anchored in the cell membrane by a transmembrane hydrophobic domain. cDNA encoding this form of LFA-3 has been cloned and sequenced (see, e.g., Wallner et al., J. Exp. Med., 166, pp. 923-932 (1987)). Another form of LFA-3 is anchored to the cell membrane via a covalent linkage to phosphatidylinositol ("PI")-containing glycolipid. This latter form has been designated "PI-linked LFA-3", and cDNA encoding this form of LFA-3 has also been cloned and sequenced (Wallner et al., PCT publication WO 90/02181).

Human LFA-3 appears to be highly homologous to the T11 target structure (T11TS) found on sheep red blood cells (SRBCs), since the natural receptor molecule of LFA-3, i.e., CD2, has been shown to bind to T11TS and human LFA-3 in a highly specific manner and with similar affinity (Selvaraj et al., J. Immunol., 139, pp. 2690-2695 (1987)). That LFA-3 and T11TS share some determinants is also reflected by the finding that a polyclonal antiserum raised against T11TS cross-reacts with human LFA-3 on human red blood cells (HRBCs) to inhibit rosetting of HRBCs with T-lymphocytes (Tiefenthaler et al., J. Immunol., 139, pp. 2696-2701 (1987)).

The LFA-3 surface protein was first characterized prior to its successful isolation and cloning by a monoclonal antibody recognizing it, designated TS2/9 (Sanchez-Madrid et al., Proc. Natl. Acad. Sci. USA, 79, pp. 7489-7493 (1982)). However, a single MAb is only of limited use in defining the various domains of LFA-3. Indeed, TS2/9 does not react with T11TS, which, as discussed above, apparently shares enough epitopes with LFA-3 to be specifically bound by CD2 (Selvaraj et al., J. Immunol., 139, pp. 2690-2695 (1987)). Antibodies recognizing different epitopes of LFA-3 than TS2/9 would be useful in studying the conformation of the protein as it is expressed in vivo. There is a need for additional antibodies that are distinct from known anti-LFA-3 antibodies, e.g., antibodies that recognize a different epitope of LFA-3 or block adhesion of leukocytes more efficiently. Such new antibodies would be useful as alternative or even preferred therapeutics to existing antibodies in the treatment of diseases that require successful binding between LFA-3 and its receptor, CD2. Such antibodies also would be useful in further studying and mapping the antigenic epitopes of the LFA-3 protein and would be useful in diagnosing LFA-3-mediated phenomena or in labeling or isolating LFA-3-expressing (LFA-3[+]) cells.

The aforementioned need is addressed by this invention, which provides new monoclonal antibodies recognizing LFA-3. More particularly, it is an object of this invention to provide monoclonal antibodies recognizing distinct epitopes of LFA-3 other than those recognized by previously described anti-LFA-3 antibodies.

BRIEF DESCRIPTION OF THE DRAWINGS

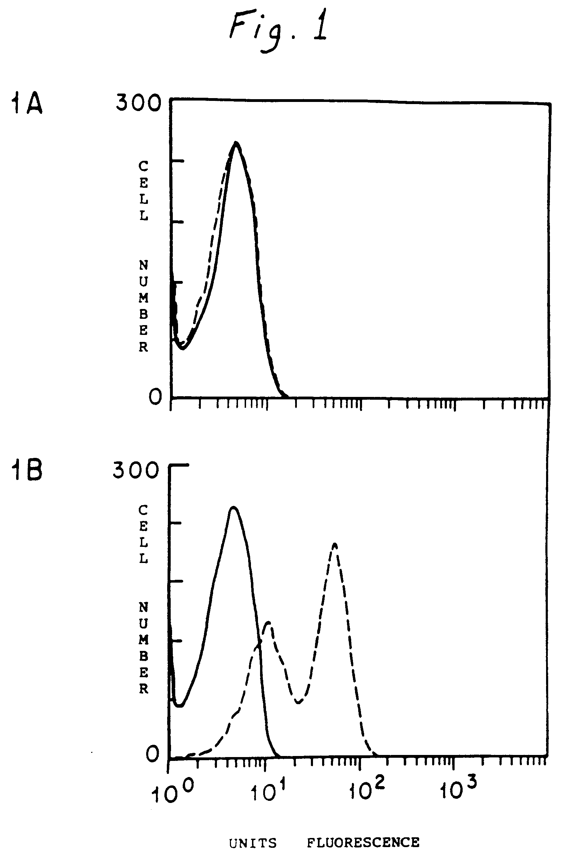

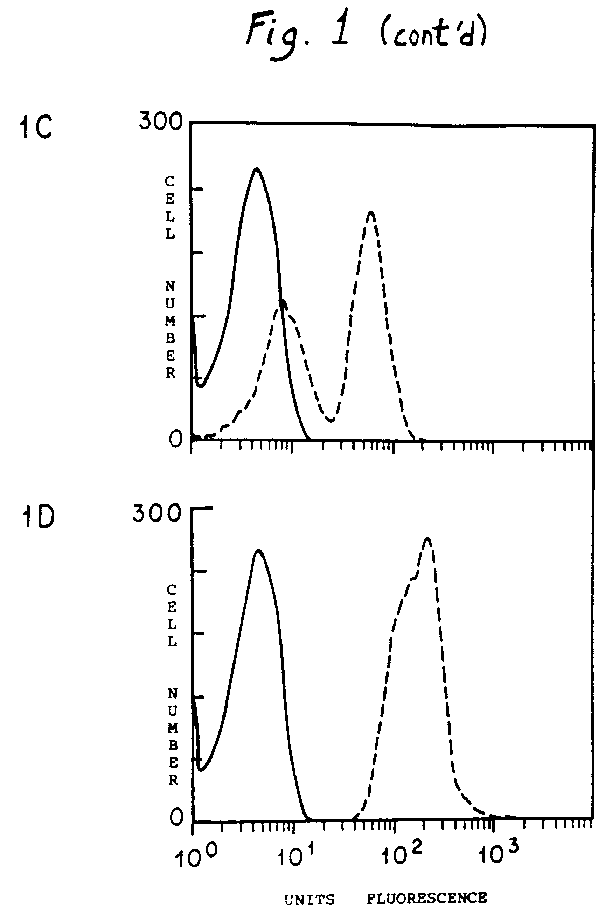

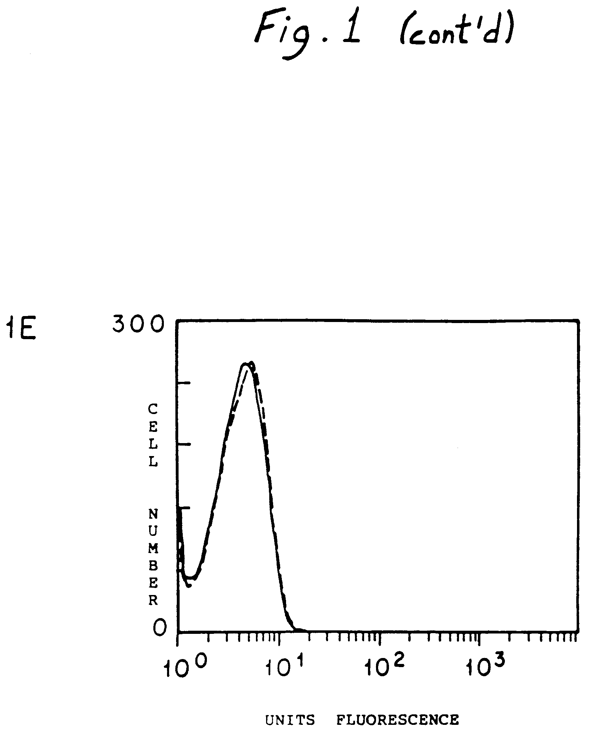

Figure 1 (Figs. 1A, 1B, 1C, 1D and 1E taken together) depicts results of immunofluorescence flow cytometry by FACS analysis of SRBCs (expressing T11TS, the LFA-3 homologue in sheep) exposed to buffer (solid lines) or to anti-LFA-3 antibodies (dashed lines) TS2/9 (Fig. 1A), 8B8 (Fig. 1B), 7A6 (Fig. 1C), HC-1B11 (Fig. 1D) or 1E6 (Fig. 1E). In each analysis, the control peak (solid line) represents the population of unbound fluorescein isothiocyanate-conjugated (FITC) goat anti-mouse IgG.

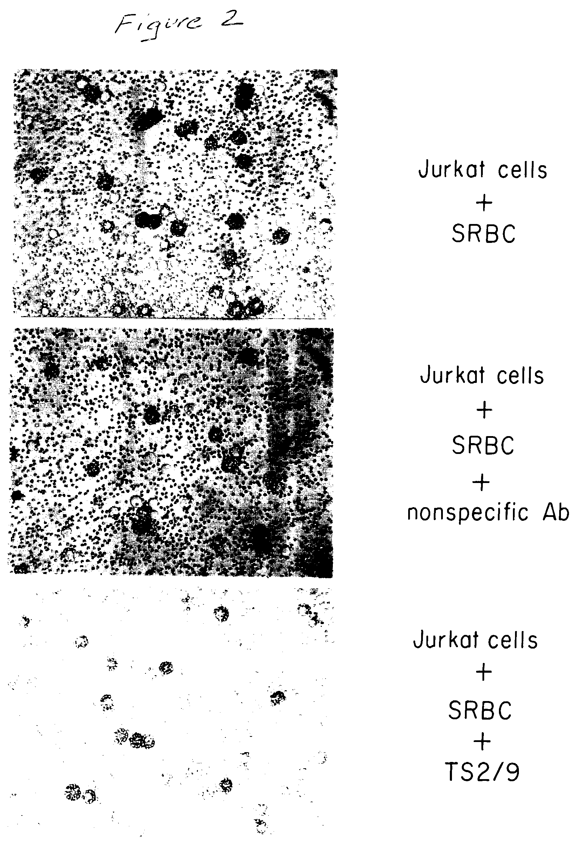

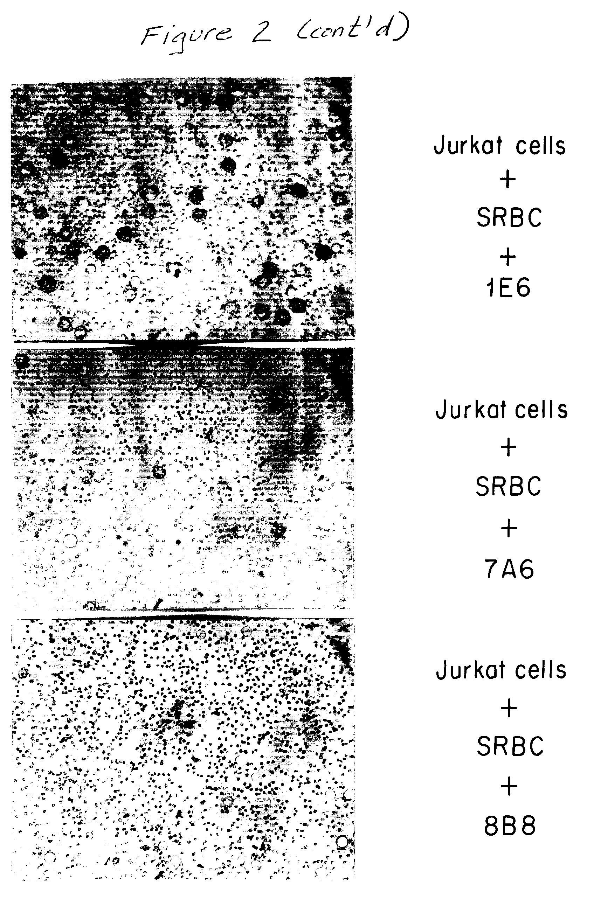

Figure 2 (Figs. 2A, 2B, 2C, 2D, 2E, and 2F taken together) depicts the results of a Jurkat cell binding experiment. The photomicrographs show Jurkat cells expressing CD2 mixed with sheep red blood cells (SRBCs) alone (positive control, maximal rosetting) (Fig. 2A), SRBCs and murine myeloma immunoglobulin (positive control, non-specific IgG1 antibody) (Fig. 2B), SRBCs and anti-LFA-3 MAb TS2/9 (Fig. 2C), SRBCs and anti-LFA-3 MAb 1E6 (Fig. 2D), SRBCs and anti-LFA-3 MAb 7A6 (Fig. 2E), and SRBCs and anti-LFA-3 MAb 8B8 (Fig. 2F).

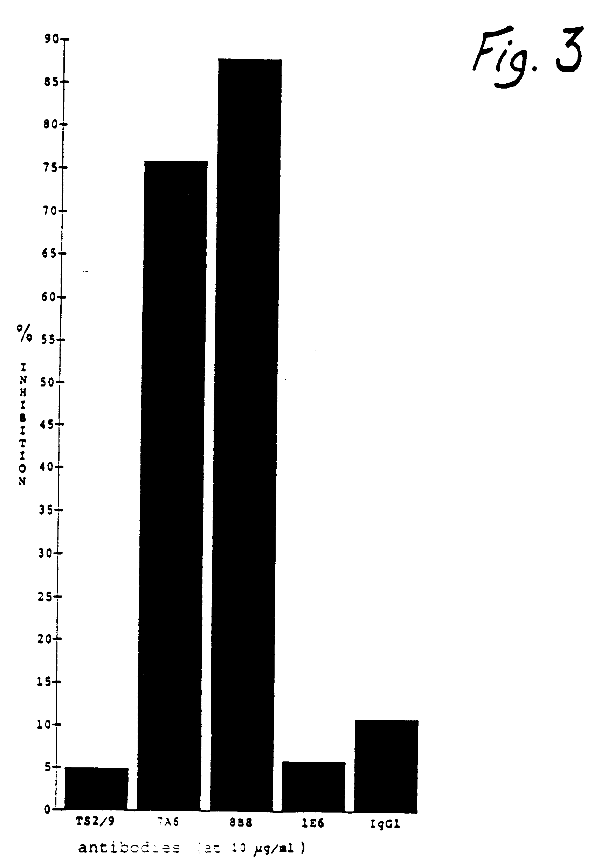

Figure 3 is a bar graph comparing percent inhibition of Jurkat/SRBC binding of different purified monoclonal anti-LFA-3 antibodies at 10 $\mu$g/ml of antibody.

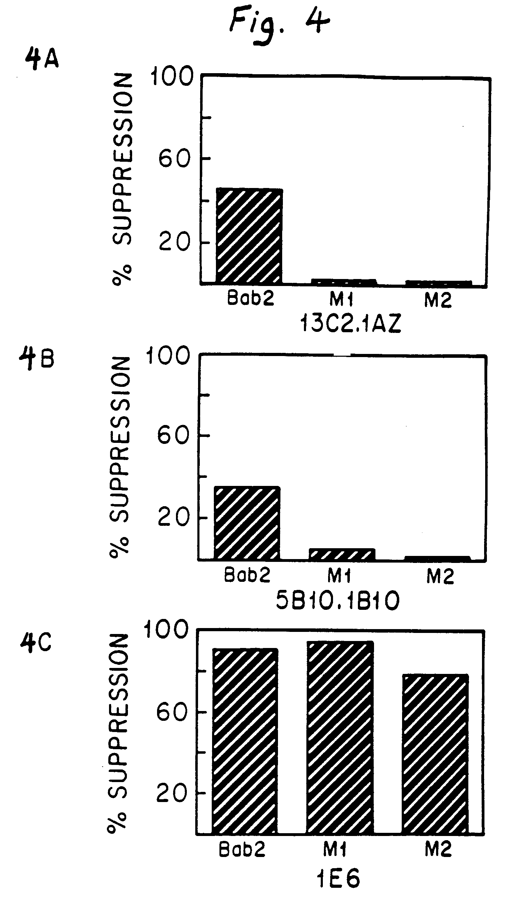

Figure 4 (Figs. 4A, 4B, and 4C) represents MLR results obtained when baboon PBLs are incubated with indicated stimulator cells. Responder cells are from a first baboon, stimulator cells are either from a second baboon (Bab2) or from cynomolgus monkeys 1 (M1) or 2 (M2). Figures 4A and 4B depict results obtained when TS2/9-like anti-LFA-3 MAbs 13C2.1AZ or 5B10.1B10 are added to the incubation. Figure 4C shows results obtained with MAb 1E6.

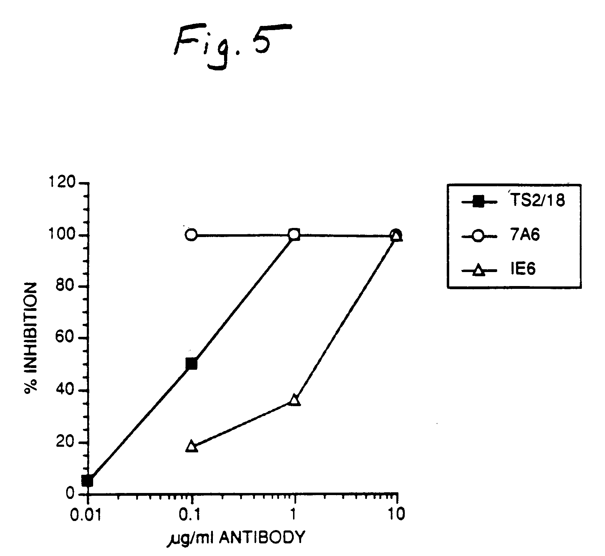

Figure 5 is a graph comparing percent inhibition of Jurkat/HRBC rosetting by TS2/18 (an anti-CD2 MAb) and by anti-LFA-3 MAbs 7A6 and 1E6 at the indicated concentrations.

The technology for producing monoclonal antibodies is well known. Briefly, an immortal cell line (typically myeloma cells) is fused to lymphocytes (typically splenocytes) from a mammal immunized with, e.g., whole cells expressing a given antigen, e.g., LFA-3, and the culture supernatants of the resulting hybridoma cells are screened for antibodies against the antigen. See, generally, Kohler et al., Nature, 256, pp. 495-497 (1975). Alternatively, immunization can be accomplished using a soluble antigen, e.g., recombinant soluble LFA-3, instead of whole cells.

Immunization may be accomplished using standard procedures. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, anti-LFA-3 antibodies were identified by testing the ability of the immune serum to block SRBC rosetting of Jurkat cells, which naturally express the LFA-3 receptor, CD2. The lymphocytes used in the production of hybridoma cells typically are isolated from immunized mammals whose sera have already tested positive for the presence of anti-LFA-3 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium").

Typically, HAT-sensitive mouse myeloma cells are fused to mouse splenocytes using polyethylene glycol ("PEG") 3350. Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce anti-LFA-3 antibodies were screened for their ability to bind to transfected cells expressing recombinant LFA-3 and for their ability to block Jurkat cell adhesion to SRBCs expressing the sheep LFA-3 homologue, T11TS. Subcloning of the hybridoma cultures by limiting dilution is typically performed to ensure monoclonality.

To produce anti-LFA-3 antibodies, hybridoma cells that tested positive in such screening assays were cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the anti-LFA-3 antibodies optionally further purified by well-known methods.

Alternatively, the desired antibody may be produced by injecting the hybridoma cells into the peritoneal cavity of a pristane-primed mouse. The hybridoma cells proliferate in the peritoneal cavity, secreting the antibody, which accumulates as ascites fluid. The antibody may be harvested by withdrawing the ascites fluid from the peritoneal cavity with a syringe.

One anti-LFA-3 monoclonal antibody (α-LFA-3 MAb) has been previously described, i.e., TS2/9 (Sanchez-Madrid et al., Proc. Natl. Acad. Sci. USA, 79, pp. 7489-7493 (1982)). In the experiments described herein, TS2/9 (gift of T. Springer, Dana Farber Cancer Institute, Boston, MA) was used for comparison.

It will also be recognized that for the purposes of the present invention the anti-LFA-3 antibodies must recognize the LFA-3 antigen and be effective to block T-lymphocyte binding to LFA-3 or to inhibit formation of the CD2/LFA-3 complex or an analogous complex, e.g., CD2/T11TS, or be effective to differentially inhibit xenogeneic or allogeneic mixed lymphocyte reaction (MLR).

Monoclonal antibodies having the characteristics described herein may alternatively be produced via recombinant DNA techniques. Such recombinant antibodies include those produced by a host cell transformed with a suitable expression vector containing DNA encoding the light and heavy immunoglobulin chains of the desired antibody; complementarity determining region (CDR) grafted antibodies (i.e., humanized antibodies); and recombinant chimeric antibodies (wherein some or all of the hinge and constant regions of the heavy and/or the light chains of the anti-LFA-3 antibody have been substituted with corresponding regions of an immunoglobulin light or heavy chain of a mammal of a different species). (See, e.g., Jones et al., "Replacing the Complementarity-Determining Regions in a Human Antibody with Those From a Mouse", Nature, 321, pp. 522-525 (1986); Ward et al., "Binding Activities of a Repertoire of Single Immunoglobulin Variable Domains Secreted From Escherichia coli", Nature, 341, pp. 544-46 (1989); U.S. Patent No. 4,816,397, Boss et al., "Multichain Polypeptides Or Proteins And Processes For Their Production", March 28, 1989; and European patent publication 0 239 400; all incorporated herein by reference.)

Recombinant antibodies may, for example, be produced by cloning cDNA or genomic DNA encoding the immunoglobulin light and heavy chains of the desired antibody from a hybridoma cell that produces a MAb of this invention. The cDNA or genomic DNA encoding those polypeptides is then inserted into expression vectors so that both DNA sequences (i.e., one DNA sequence encoding the light chain and another DNA sequence encoding the heavy chain) are operatively linked to one or more transcriptional and translational expression control sequences. The expression vector and expression control sequences are chosen to be compatible with the expression host cell used. Typically, both DNA sequences are inserted into the same expression vector, although the two DNA sequences may also be inserted into different expression vectors.

One such recombinant antibody of this invention, 1E6, is directed against the CD2-binding domain of LFA-3. The isolated DNA sequences provided herein encode the variable region of the 1E6 light chain (SEQ ID NO:1), and the variable region of the 1E6 heavy chain (SEQ ID NO:3). Also provided are DNA sequences degenerate to SEQ ID NO:1 and SEQ ID NO:3, as well as portions of SEQ ID NO:1 and SEQ ID NO:3, and sequences degenerate thereto, which encode polypeptides that bind, by themselves or in combination with other polypeptides, to LFA-3 at a different epitope than that recognized by MAb TS2/9. The present invention further provides hybrid DNA sequences comprising one or more of the foregoing DNA sequences fused to DNA sequences which do not encode any portion of 1E6.

Also within the scope of this invention are recombinant DNA molecules comprising one or more of the foregoing DNA sequences and one or more expression control sequences operatively linked thereto.

Prokaryotic and eukaryotic cells may be used as expression hosts. Expression in eukaryotic host cells is preferred because such cells are more likely to assemble and secrete a properly folded and immunologically active antibody. However, an antibody produced using the DNA sequences of this invention that is inactive because of improper folding may be renatured using well known methods (see, e.g., Kim and Baldwin, "Specific Intermediates In The Folding Reactions Of Small Proteins And The Mechanisms Of Protein Folding", Ann. Rev. Biochem., 51, pp. 459-489 (1982)). It is also possible that the host cells will produce portions of intact antibodies according to this invention, such as light chain dimers or heavy chain dimers.

It will be understood that variations on the above procedures are within the scope of the present invention. For example, it may be desired to transform a host cell with DNA encoding either the light chain or the heavy chain (but not both) of an antibody of this invention. Recombinant DNA technology may also be used to remove some or all of the DNA encoding either or both of the light and heavy chains that is not necessary for LFA-3 binding. In addition, bifunctional antibodies may be produced in which one heavy and one light chain are derived from an antibody of this invention, and are specific for one epitope of LFA-3, and the other heavy and light chain are specific for an antigen other than LFA-3, or for a different epitope of LFA-3.

DNA encoding the recombinant antibodies described herein may be used to produce chimeric or humanized (CDR-grafted) recombinant antibodies. Chimeric recombinant antibodies are produced by transforming a host cell with a suitable expression vector comprising DNA encoding the desired immunoglobulin light and heavy chains in which all or some of the DNA encoding the hinge and constant regions of the heavy and/or light chain have been substituted with DNA from the corresponding region of an immunoglobulin light or heavy chain of a mammal of a different species. When the chimeric antibody is for use in humans and the original recombinant antibody is nonhuman, substitution of corresponding human sequences is preferred. An example of a chimeric recombinant antibody has mouse variable regions and human hinge and constant regions. See generally, United States Patent 4,816,397 and Morrison et al., "Chimeric Human Antibody Molecules: Mouse Antigen-Binding Domains With Human Constant Region Domains", Proc. Natl. Acad. Sci. USA, 81, pp. 6851-6855 (1984).

A preferred chimeric recombinant antibody of this invention has (SEQ ID NO:2) as the light chain variable region and (SEQ ID NO:4) as the heavy chain variable region.*

Humanized recombinant antibodies according to this invention are produced by transforming a host cell with a suitable expression vector comprising DNA encoding the desired nonhuman immunoglobulin light and heavy chains in which all or some of the DNA encoding amino acids not involved in LFA-3 binding have been substituted with DNA from the corresponding region of a desired human immunoglobulin light or heavy chain (see, e.g., Jones et al., supra, 1986) and EP 0 239 400).

This invention provides the DNA sequences encoding the specific complementarity determining regions (CDRs) of the light and heavy chain variable regions of MAb 1E6. The identification of the CDRs of the variable region of the light and heavy chains of 1E6 enables one of skill in the art to produce humanized recombinant antibodies, as mentioned above. Preferred humanized recombinant antibodies of this invention are capable of binding to LFA-3 and are selected from the group consisting of humanized recombinant antibodies wherein:

(1) light chain CDR1 is $AA_{24}$-$AA_{34}$ of SEQ ID NO:2,
(2) light chain CDR2 is $AA_{50}$-$AA_{56}$ of SEQ ID NO:2,
(3) light chain CDR3 is $AA_{59}$-$AA_{97}$ of SEQ ID NO:2,
(4) heavy chain CDR1 is $AA_{31}$-$AA_{35}$ of SEQ ID NO:4,
(5) heavy chain CDR2 is $AA_{50}$-$AA_{56}$ of SEQ ID NO:4,
and
(6) heavy chain CDR3 is $AA_{99}$-$AA_{108}$ of SEQ ID NO:4.

It will be understood that it may be desirable to substitute one or more amino acids near the junctures of the CDRs and framework regions (FRs) of the humanized antibody with other amino acids in order to increase affinity for LFA-3 (see, e.g., European patent publication 0 239 400).

LFA-3-binding fragments of anti-LFA-3 antibodies, such as Fab, Fab', $F(ab)_2$, and F(v) fragments; heavy chain monomers or dimers; light chain monomers or dimers; and dimers consisting of one heavy chain and one light chain are also contemplated herein. Such antibody fragments may be produced by chemical methods, e.g., by cleaving an intact antibody with a protease, such as pepsin or papain, or via recombinant DNA techniques, e.g., by using host cells transformed with truncated heavy and/or light chain genes. Heavy and light chain monomers may similarly be produced by treating an intact antibody with a reducing agent such as dithiothreitol or $\beta$-mercaptoethanol or by using host cells transformed with DNA encoding either the desired heavy chain or light chain or both.

Another alternative to hybridoma technology is the use of phage cloning methods (see, e.g., Clackson et al., Nature, 352, pp. 624-628 (1991)) to produce polypeptides having similar specificities to the antibodies of this invention.

The anti-LFA-3 antibodies of the present invention are effective to block adhesion between the T-lymphocyte-like cell line, Jurkat (a gift from T. Springer, Dana Farber Cancer Institute, Boston, MA), and LFA-3[+] cells, such as P24/CHO or P24neoR1.1 cells (i.e., CHO cells or R1.1 cells transfected with plasmid P24, coding for PI-linked LFA-3, described in PCT publication WO 90/02181). However, they recognize an epitope distinct from that recognized by MAb TS2/9.

The monoclonal antibodies of the present invention may be used in any application where antibody recognition of LFA-3 is advantageous, and particularly in applications where inhibiting binding between LFA-3 and its receptor (CD2) is desired. For example, the monoclonal antibodies of the present invention may be used in the treatment of acute and chronic inflammation, autoimmune diseases, and for immunomodulation, including such diseases as systemic lupus erythematosus or lupus vulgaris, rheumatoid arthritis and thyroiditis.

* This sequence is missing the first amino acid of the mature heavy chain variable region. The skilled person easily can determine the identity of that amino acid or of an acceptable substitute.

The antibodies of the present invention may also be used in combination with other antibodies, bioactive agents or materials for various purposes. For example, the present antibodies may be used in combination with TS2/9 or other anti-LFA-3 antibodies in the treatment of disorders characterized by, or dependent on, CD2/LFA-3 binding. Alternatively, the present monoclonal antibodies may be used in combination with anti-CD2 antibodies to augment or inhibit immune responses, or with antibodies recognizing other T-lymphocyte cell receptors for, e.g., promoting T-lymphocyte proliferation or otherwise augmenting the immune response. Also, the antibodies of the present invention, or LFA-3-recognizing fragments thereof, may be combined or linked to cytotoxic molecules such as TNF, ricin or the A chain of diphtheria toxin, in order to provide antibody/toxin conjugates capable of targeting cytotoxic elements to LFA-3-expressing cells. The antibodies of the present invention may also be immobilized on a chromatographic substrate (e.g., protein A-Sepharose) to provide an affinity chromatography resin useful for separating or purifying LFA-3.

Detectably labeled antibodies according to the present invention may also be used in screening methods or diagnostic methods for detecting LFA-3 protein in a sample or LFA-3-expressing cells in vitro or in vivo, or for characterizing disease events that are interrelated with regulation of LFA-3 expression. For example, samples may be screened for the presence of LFA-3 or LFA-3$^+$ cells by contacting the sample with a labeled antibody according to the present invention and detecting whether an LFA-3/anti-LFA-3 antibody complex is formed.

Suitable labels can be radioactive, enzymatic, fluorescent, magnetic or chemiluminescent. Radiolabeled antibodies are prepared in known ways by coupling a radioactive isotope such as $^3$H, $^{32}$P, $^{35}$S, $^{59}$Fe or $^{125}$I, which can then be detected by gamma counter, scintillation counter or by autoradiography. Antibodies of this invention may be suitably labeled with enzymes such as yeast alcohol dehydrogenase, horseradish peroxidase, alkaline phosphatase, and the like, then developed and detected spectrophotometrically or visually. Suitable fluorescent labels include fluorescein isothiocyanate, fluorescamine, phycoerythrin ("PE"), rhodamine, and the like. Suitable chemiluminescent labels include luminol, imidazole, oxalate ester, luciferin, and the like.

For therapeutic uses, the monoclonal antibodies of the present invention may be formulated as a pharmaceutical composition comprising an effective amount of the antibody admixed with a pharmaceutically acceptable carrier. Typically, the antibodies of the present invention will be suspended in a sterile saline solution for therapeutic uses. The pharmaceutical compositions may alternatively be formulated to control release of the active ingredients or to prolong their presence in a patient's system. Numerous suitable drug delivery systems are known and include, e.g., hydrogels, hydroxymethylcellulose, microcapsules, liposomes, microemulsions, microspheres, and the like.

The pharmaceutical compositions contemplated herein may be administered by any suitable means, such as orally, intranasally, subcutaneously, intradermally, intramuscularly, intravenously, intra-arterially, or parenterally. Ordinarily, intravenous (i.v.) or parenteral administration will be preferred, however more localized administrations may be more desirable in some cases due to the wide range of cells in the body that express LFA-3.

The Examples illustrate the invention.

## EXAMPLE 1

### Preparation of Hybridomas

Hybridomas secreting the MAbs of this invention were selected from a population of fused cells formed by fusing mouse myeloma P3/X63-Ag8.653 cells (available from American Type Culture Collection (ATCC), Rockville, Maryland, under ATCC accession no. CRL 1580) to a pooled population of splenic lymphocytes isolated from two BALB/c mice immunized with HT16/CHO, i.e., CHO cells transfected with BG8 -- an animal cell expression vector encoding transmembrane LFA-3. Microorganisms bearing vector BG8 were deposited with American Type Culture Collection (ATCC accession number 68791). The isolation of cDNA encoding transmembrane LFA-3 has been described in U.S. Patent 4,956,281 to Wallner et al., and a bacteriophage bearing HT16 cDNA coding for transmembrane LFA-3, λHT16[λgt10/LFA-3], was deposited with American Type Culture Collection (ATCC accession number 75107). The production of HT16/CHO cells is described in PCT patent application WO 88/09820, which is herein incorporated by reference.

One mouse was primed initially with approximately $2 \times 10^6$ HT16/CHO cells in PBS intravenously (i.v.) and boosted after sixteen days with $2 \times 10^6$ HT16/CHO cells in PBS intraperitoneally (i.p.). The other mouse was primed with approximately $2 \times 10^6$ HT16/CHO cells 1:1 in Freund's complete adjuvant i.p. and boosted after twenty-five days i.p. with $2 \times 10^6$ HT16/CHO cells in PBS. Both mice were boosted a second time, i.p.,

with $1 \times 10^6$ HT16/CHO cells in PBS after forty-six and twenty-one days, respectively, three days before fusion. Hybridomas were generated from polyethylene glycol-mediated fusion of spleen lymphocytes from the immunized mice (see above) and mouse myeloma P3/X63-Ag8.653 cells according to the procedure of Lerner (Yale J. Biol. Med., 54, pp. 387-402 (1981)). The fusion partner in all cases was mouse myeloma P3/X63-Ag8.653 cells, and the ratio of lymphocytes to myeloma cells for the fusion procedure was approximately 5:1.

EXAMPLE 2

Primary Screen For Hybridomas Producing Anti-LFA-3 Monoclonal Antibodies by Radioimmunoassay

The initial screening protocols were used to insure that hybridomas producing antibodies specific for recombinant or natural LFA-3 would be detected. In the primary screen, the supernatants of cultures of individual hybridoma clones were first screened for the presence of anti-LFA-3 MAbs by determining whether the antibodies in the culture supernatants would bind to murine P24neoR1.1 cells (which express PI-linked LFA-3) but not to R1.1 cells (which do not express LFA-3), using a radioimmunoassay ("RIA"). The P24neoR1.1 cells are R1.1 cells (ATCC T1B42) transfected with the recombinant expression plasmid P24, which is described in PCT publication WO 90/02181.

Approximately $1.25 \times 10^5$ cells in 50 $\mu$l 1X PBS with 1% BSA was combined with 50 $\mu$l of hybridoma culture supernatant in wells of a 96-well microtiter dish, and incubated for 1 hour at 4°C. The cells were then pelleted to the bottom of the wells and washed three times with PBS$^=$/BSA/Azide buffer (phosphate buffered saline lacking Ca$^{++}$ and Mg$^{++}$; 1% BSA; 0.1% azide) to remove unbound antibodies.

The washed cells were then mixed with goat anti-mouse $^{125}$I-labeled IgG secondary antibody (approximately $5 \times 10^4$ cpm, New England Nuclear) and incubated for 1 hour at 4°C. After incubation, the cells were washed three times with PBS$^=$/BSA/Azide buffer to remove unbound labeled antibodies, and the wells cut out of the microtiter dish for counting in a gamma counter.

Secondary Screen for Hybridomas Producing Anti-LFA-3 Monoclonal Antibodies by FACS Analysis

Clones that, according to the RIA primary screening assay, produced MAbs that bound to the P24neoR1.1 cells but not untransfected R1.1 cells, were screened a second time in an immunofluorescence assay using a fluorochrome conjugated anti-mouse reagent in a fluorescence-activated cell sorter analysis ("FACS") to test the ability of the MAbs in the supernatants to bind to naturally produced LFA-3 on the surface of JY $\beta$-lymphoblastoid cells.

For this second screening of the hybridoma culture supernatants, approximately $1.25 \times 10^5$ JY cells (a gift of T. Springer, Dana Farber Cancer Institute, Boston, Massachusetts) were mixed with an equal volume (50 $\mu$l) of culture supernatant and incubated for 1 hour at 4°C. The cells were then washed three times with PBS$^=$/1% BSA/0.1% Sodium Azide buffer and resuspended in the same buffer.

The cell suspension was then mixed with PE-labeled goat anti-mouse IgG, and the mixtures incubated for 1 hour at 4°C. The cells were then washed twice with PBS$^=$/BSA/Azide buffer and then once with PBS/Azide buffer (same as PBS$^=$/BSA/Azide but lacking BSA). If the labeled cells were not to be used for FACS analysis immediately, they were fixed by adding to the cell suspension an equal volume of 2% formalin and stored at 4°C until used, typically the following day.

Of the original 803 hybridomas isolated from the fusions described above, 23 clones (3%) gave positive results in the primary (RIA) and secondary (FACS) screens and were presumed to produce MAbs specific for LFA-3.

EXAMPLE 3

Determination of Anti-LFA-3 Specificity of MAbs by Additional Functional Assays

Anti-LFA-3 specificity and the nature of epitope recognition for the MAbs produced by the 23 positive hybridoma clones according to the primary and secondary screens (see supra) were explored further in several functional assays, i.e., a cytotoxic T-lymphocyte ("CTL") killing assay employing JY cells, a mixed lymphocyte reaction ("MLR") assay, and an alloresponse assay of peripheral blood lymphocytes ("PBLs") to JY tumor cells. Formation of the CD2/LFA-3 complex plays a significant role in all three of these interactions. Thus, inhibition of any of these assays by MAbs produced by any of the hybridoma clones was indicative of specificity for the LFA-3 molecule and, in particular, regions of LFA-3 involved in the binding of CD2.

## Inhibition of CTL-Mediated Killing of JY Cells

A cytotoxic T-lymphocyte ("CTL") assay was originally used to document the activity of anti-CD2 and anti-LFA-3 MAbs (Sanchez-Madrid et al., supra, 1982). In the CTL assay used to characterize the MAbs of this invention, the anti-LFA-3 MAb TS2/9 (Sanchez-Madrid et al., supra, 1982), was used as a positive control.

The CTLs used in this assay were cells of a $CD2^+$ human CTL clone designated 8.2 specific for an allogeneic class II molecule (HLA-DPw2), obtained as a gift from S. Shaw (NIH). Cells of the human lymphoblastoid cell line, JY, were used as the LFA-3 target cells. Typically, $1 \times 10^4$ CTLs were preincubated with 50 $\mu$l of hybridoma culture supernatants for 30 minutes at room temperature (25°C) prior to addition of $2 \times 10^3$ JY target cells. A standard $^{51}$Cr-release assay (see, e.g., Russel et al., J. Immun., 128, pp. 2087-2094 (1982)) was performed in 96-well V-bottomed plates to determine the extent of killing of JY cells. The percent killing was monitored over time, and the assay was stopped when the percentage of cells killed reached approximately 50%.

The results of the CTL killing assays indicated that TS2/9 and the MAbs in the hybridoma culture supernatants inhibited CTL-mediated killing of JY cells, confirming the anti-LFA-3 specificity of the MAbs. However, since it was supernatants of the various hybridomas that were used in this assay and not purified MAbs, any quantitative difference between any of the MAbs or between the MAbs and TS2/9 could not be determined.

## Inhibition of Mixed Lymphocyte Reaction

Another functional assay for the formation of the CD2/LFA-3 complex is the mixed lymphocyte reaction ("MLR") (see, e.g., Krensky et al., J. Immunol., 131(2), pp. 611-616 (1983); Bradley, "Mixed Lymphocyte Responses", in Selected Methods in Cellular Immunology (Mishell and Shiigi, eds.), pp. 162-164 (W.H. Freeman and Co., San Francisco 1980). This assay is based on the activation of T-lymphocytes in a population of peripheral blood lymphocytes ("PBLs") when the T-lymphocytes recognize alloantigens in nonproliferating allogeneic PBLs. Such activation occurs due to cell-cell adhesion mediated in part by the binding of CD2 molecules on the T-lymphocytes to the LFA-3 molecules on the allogeneic PBLs.

PBLs from blood taken from two allogeneic human donors were separated on Ficoll-Paque (Pharmacia) according to the manufacturer's specifications. The PBLs from one of the donors were also irradiated (2000 rads) to destroy proliferative activity. Equal numbers ($1 \times 10^5$ cells) of the nonirradiated and irradiated PBLs in 50 $\mu$l of culture medium (RPMI 1640 (Gibco) supplemented with 10% fetal bovine serum, 1X penstrep (100 units/ml penicillin G, 10 $\mu$g/ml streptomycin, Whittaker Bioproducts, Inc.) and L-glutamine (2 mM in 0.0085% NaCl)) were added to round bottomed wells of a 96-well microtiter plate. Culture medium or antibody preparations were then added to bring the final volume in each well to 200 $\mu$l. The mixed lymphocyte cultures were incubated for 6 days at 37°C, and 50 $\mu$l of culture medium containing 1 $\mu$Ci of $^3$H-thymidine (New England Nuclear, Boston, MA) was added to each culture for the final 6 hours of incubation. Proliferation of the nonirradiated population of T-lymphocytes was measured by determining incorporation of the $^3$H-thymidine into the cells, indicating uptake of the labeled thymidine during T-lymphocyte proliferation.

## Inhibition of Alloresponse

Another functional assay used to test for the presence of anti-LFA-3 MAbs in supernatants of cultures of hybridomas testing positive in the primary and secondary screens (see supra), was a standard inhibition of alloresponse assay (see, e.g., Krensky et al., J. Immun., 135(5), pp. 3102-3108 (1985)). This assay is similar to the MLR assay above but tests for the inhibition of PBLs of one allogeneic donor to respond to irradiated (nonproliferating) JY cells.

PBLs were prepared as described above. JY cells were irradiated with 10,000 rads basically as described above. PBLs ($1 \times 10^5$) were combined with JY cells ($1 \times 10^4$) in wells of a microtiter dish, and incubated and assayed for $^3$H-thymidine incorporation as described above for the MLR assay. The results of the alloresponse assay indicated that clones which inhibited the MLR (supra) also produced MAbs which inhibited this alloresponse assay.

MAbs from culture supernatants of six hybridoma clones, which gave positive results in the primary and secondary screens and also exhibited the ability to inhibit CTL killing of JY cells, the mixed lymphocyte reaction and the alloresponse assay, were further characterized (see infra) and found to recognize epitopes on LFA-3 distinct from the epitope recognized by the anti-LFA-3 MAb TS2/9 of Sanchez-Madrid et al. (Sanchez-Madrid et al., supra, (1982)).

EXAMPLE 4

Heterologous Erythrocyte Binding by MAbs

Anti-LFA-3 MAbs, including novel MAbs 7A6, 8B8 and HC-1B11, in supernatants from cultures of the hybridoma clones were also tested for the ability to bind (i.e., "stain") sheep red blood cells (SRBCs) as determined by FACS analysis.

Human CD2 of T-lymphocytes is known to bind to the T11 target structure on SRBCs (in heterologous binding) and to LFA-3 molecules on HRBCs (in the case of autologous binding) with similar affinity, accounting for the well-known property of T-lymphocytes to form rosettes with SRBCs or bromelain-treated HRBCs (Selvaraj et al., supra, 1987). Since anti-CD2 antibodies have been shown to block the binding of T-lymphocytes to both SRBCs and HRBCs, T11TS and LFA-3 are assumed to be highly homologous. Anti-LFA-3 antibodies might therefore bind SRBCs. Accordingly, we compared the ability of our MAbs and the anti-LFA-3 MAb TS2/9 to bind SRBCs.

SRBCs (Colorado Serum Corp., Denver, Colorado) were plated into a 96-well U-bottom plate at approximately $2 \times 10^6$ cells per well. The buffer used for the washes and dilutions was PBS/BSA (1%)/azide (0.1%) and all incubations were done at 4°C. The cells were washed once with 100 $\mu$l of PBS/BSA/azide by centrifugation at approximately 1500 rpm for 5 minutes. The wash was discarded and the pellets were resuspended in 100 $\mu$l of antibody supernatant (neat) for an incubation of 1 hour at 4°C. After 1 hour, the cells were pelleted and washed three times with 100 $\mu$l PBS/BSA/azide as above. Following the last wash, the pellets were resuspended in 50 $\mu$l of fluorescein labeled goat anti-mouse IgG (H+L) FITC (at a 1:10 dilution in PBS/BSA/azide) and incubated for approximately 30 minutes at 4°C. Included as controls were SRBCs with buffer instead of culture supernatant (to show no background staining of FITC conjugation on SRBCs). After 30 minutes, the cells were washed twice with 100 $\mu$l PBS/BSA/azide and once with 100 $\mu$l PBS/azide. Finally, the pellets were resuspended in 400 $\mu$l PBS/azide and analyzed on a FACSTAR cell sorter for fluorescence.

Referring to Figure 1, the results of FACS analysis of the SRBC staining experiments clearly indicate that the anti-LFA-3 MAb TS2/9 does not bind SRBCs (Fig. 1A), whereas the anti-LFA-3 MAbs 8B8, 7A6 and HC-1B11 do bind SRBCs (Figs. 1B, 1C, 1D, respectively).

EXAMPLE 5

Inhibition of Rosette Formation by MAbs

The ability of T-lymphocytes to bind SRBCs, thereby forming characteristic rosettes, is known to be mediated by the binding of CD2 molecules on the surface of T-lymphocytes to T11TS molecules present on the surface of SRBCs (Selvaraj et al., supra, (1987)). Since anti-LFA-3 MAbs 7A6, 8B8 and HC-1B11 were found to bind SRBCs, in contrast to TS2/9, these MAbs were also tested for the ability to inhibit T-lymphocyte binding to SRBCs.

Jurkat cells, which express CD2, and SRBCs (Colorado Serum Corp., Denver, Colorado) were each washed and resuspended in culture medium (RPMI 1640, 10% fetal calf serum, 1% polyethylene glycol (PEG 8000)). Incubations were carried out in V-bottom 96-well microtiter plates (Dynatech, Alexandria, Virginia) on ice for 2 hours at a ratio of Jurkat:SRBC of about 1:100. Control samples were prepared by mixing SRBCs and Jurkat cells in medium and incubating on ice for 2 hours. Samples for testing inhibition of rosetting were prepared by pre-incubating SRBCs with purified antibody on ice for 20 minutes, then adding Jurkat cells and incubating on ice for an additional 2 hours. After incubating, cell samples were carefully transferred to a hemocytometer to count rosettes. Three or more SRBCs attached to a Jurkat target cell was considered a rosette. Rosetted Jurkat cells were quantitated as a function of total Jurkat cells present, where a value of 100% rosetting was taken from control samples containing only Jurkat cells and SRBCs (maximal rosetting).

Referring to Figure 2, rosette formation was clearly observed in the control reaction (Fig. 2A). In the presence of a murine myeloma IgG (non-specific antibody), which does not react with antigens on SRBCs, rosettes also were formed (Fig. 2B). Rosetting was not inhibited in the presence of anti-LFA-3 MAb TS2/9 (Fig. 2C) or MAb 1E6 (Fig. 2D), one of the positive-scoring antibodies from the first two screens. However, MAbs 7A6 and 8B8 both inhibited rosette formation (Figs. 2E and 2F, respectively).

Referring to Figure 3, results of SRBC rosetting inhibition assays are shown for purified monoclonal antibodies TS2/9, 7A6, 8B8, 1E6 and IgG1 (control). The data show percent inhibition attributable to each antibody at a dose level of 10 $\mu$g/ml antibody (purified from ascites). The results demonstrate differences between the anti-LFA-3 MAbs of this invention and TS2/9 in their ability to inhibit heterologous rosette formation. MAbs 7A6 and 8B8 are seen to recognize epitopes of LFA-3 that are distinct from the epitope recognized by TS2/9.

As an additional assay of epitope recognition, MAbs of this invention were tested for the ability to inhibit T-lymphocyte binding to bromelain-treated human RBCs (HRBCs).

Whole blood was taken from a healthy donor and centrifuged for 30 minutes at 1600 rpm at room temperature. The buffy coats were removed, and HRBCs were pelleted by centrifugation. The HRBCs were suspended in a solution of bromelain (1 mg/ml) in 1X PBS$^=$ (i.e., Ca$^{+2}$ and Mg$^{+2}$ free). After 30 minutes at 37°C, the HRBCs were washed twice with RPMI medium and resuspended in RPMI supplemented with 10% fetal calf serum (FCS) and 1% PEG 8000.

CD2$^+$ Jurkat T cells were washed three times with RPMI and resuspended in RPMI supplemented with FCS and PEG 8000, as above. Aliquots of Jurkat cells (1 x 10$^5$ cells/well) were dispensed into V-bottomed 96 well tissue culture plates, and incubated with MAbs TS2/18, (an anti-CD2 MAb used as a positive control (Sanchez-Madrid et al., supra, (1982)), 7A6 or 1E6 (all at 10 $\mu$g/ml), or with medium alone (control), for 20 minutes at room temperature. Bromelain-treated HRBCs (1 x 10$^7$) were then added to each well, and the plates were centrifuged at 1000 rpm at 4°C for 1 minute. The plates were then incubated on ice for 2 hours. After incubation, the cells were gently mixed and 5 $\mu$l of the suspension was transferred to 100 $\mu$l of RPMI medium in a 96 well flat bottom plate.

Rosetted Jurkat cells were counted under a microscope at 100X magnification. The percent inhibition of rosetting for each MAb was determined as for SRBC rosetting assay above (i.e., the difference between the percent of rosettes in the control well and MAb-containing wells divided by the percent of rosettes in the control well).

Referring to Figure 5, results of HRBC rosetting inhibition assays are shown for MAbs 7A6, 1E6 and TS2/18. The results show that 7A6 and 1E6 differ in their ability to inhibit rosetting of HRBCs. Also, 7A6 was clearly superior to both TS2/18 and 1E6 at inhibiting rosetting of HRBCs.

EXAMPLE 6

Allogeneic and Xenogeneic Mixed Lymphocyte Reaction:

A MLR was set up as described above, except that baboon PBLs were used as responder cells and either gamma-irradiated cynomolgus monkey PBLs (xenogeneic) or PBLS from a different baboon (allogeneic) were used as stimulator cells. The reactions were incubated as described above in RPMI medium plus 15% baboon serum. Incubations were carried out either in medium alone (control) or in the presence of anti-LFA-3 MAbs as indicated below. Two MAbs, designated 13C2.1AZ and 5B10.1B10 (determined to be TS2/9-like by cross-blocking experiments), were used for comparison against MAb 1E6 (this invention). The concentration of all MAbs in the incubation was 1 $\mu$g/ml.

Referring to Figure 4, inhibition by each antibody is stated as percent (%) suppression from the value obtained in the control incubation. The TS2/9-like MAbs 13C2.1AZ and 5B10.1B10 inhibited the allogeneic MLR ("Bab2" in Figures 4A and 4B) at 42% and 38%, respectively. No significant inhibition of the xenogeneic MLR by MAbs 13C2.1AZ and 5B10.1B10 was detected ("M1" and "M2" in Figures 4A and 4B). MAb 1E6, on the other hand, inhibited both the xenogeneic and the allogeneic MLR between 80 and 100% (Figure 4C).

EXAMPLE 7

Cloning And Sequencing Of 1E6 Light And Heavy Chain Variable Regions

Total RNA was prepared from the hybridoma cell line 1E6-2C12 (ATCC HB 10693), which produces the 1E6 MAb, according to a conventional guanidinium isothiocyanate protocol. cDNA was prepared from the total RNA as follows. The cDNA synthesis reaction contained 1 $\mu$l 1E6 RNA (~1 $\mu$g), 8 $\mu$l sterile water, 4 $\mu$l 5X reverse transcriptase buffer (250 mM Tris/HCl, pH 8.3, 375 mM KCl, 50 mM dithiothreitol (DTT), 15 mM MgCl$_2$), 2 $\mu$l 10 mM dXTPs (i.e., the four deoxynucleoside triphosphates), 1 $\mu$l M-MLV reverse transcriptase (200 U/$\mu$l, BRL, Gaithersburg, Maryland), 1 $\mu$l (100 pmol/$\mu$l) random hexamers as primers, 2 $\mu$l DTT (100

mM), 1 $\mu$l human placental ribonuclease inhibitor (20 U/$\mu$l, BRL). The reaction mixture was incubated for 30 minutes at 42°C, terminated by heating to 95°C, and then immediately stored on ice.

DNA sequences encoding the light and heavy chain variable regions of 1E6 were amplified for cloning and sequencing using polymerase chain reaction (PCR) (see, e.g., Orlandi et al., "Cloning Immunoglobulin Variable Domains For Expression By The Polymerase Chain Reaction", Proc. Natl. Acad. Sci. USA, 86, pp. 3833-3837 (1989)). Prior to amplification, 20 pmoles of each of the light chain primers (MKVP7 (SEQ ID NO:6) and 360-41 (SEQ ID NO:7)) and the heavy chain primers (VH01 (SEQ ID NO:8) and VH02 (SEQ ID NO:9)) were kinased by incubation with T4 polynucleotide kinase at 37°C for 60 minutes by a conventional protocol. The kinase reactions were stopped by heating at 70°C for 10 minutes.

The PCR reactions each contained 10 $\mu$l 10X PCR buffer (10X PCR buffer is 100 mM Tris/HCl, pH 8.3, 500 mM KCl, 15 mM MgCl$_2$, 0.01% gelatin, 20 pmoles each of the appropriate kinased primers (MKVP7 and 360-41 for light chain variable region; VH01 and VH02 for heavy chain variable region), 20 $\mu$l cDNA, 0.5 $\mu$l Taq polymerase (5 U/$\mu$l, Perkin Elmer-Cetus) and 49.5 $\mu$l H$_2$O. The PCR conditions were 30 cycles of incubation for: 1 minute at 94°C; 2 minutes at 40°C (for heavy chain PCR) or at 55°C (for light chain PCR); and 2 minutes at 72°C.

The reaction mixtures were electrophoresed through 2% agarose gel, and the bands corresponding to the expected sizes of the heavy chain variable region (~330 bp) and the light chain variable and constant regions (~700 bp) were excised. The DNA in those bands were eluted using the GENECLEAN technique (Bio101 Inc., LaJolla, California), ethanol precipitated and subsequently each resuspended in 20 $\mu$l TE buffer (10 mM Tris-HCl, 1 mM Na$_2$EDTA).

Klenow fragment of DNA polymerase (New England Biolabs, 5 U/$\mu$l) (1 $\mu$l) was added to the purified PCR fragments in a reaction volume of 25 $\mu$l containing 1x ligation buffer (10x ligation buffer is 0.5 M Tris/HCl, pH 7.5, 100 mM MgCl$_2$ and 40 mM DTT) and 0.125 mM each of dXTPs and the reaction incubated at room temperature for 15 minutes. The reaction was terminated by incubation at 70°C for 5 minutes, and then stored on ice.

The fragment from each PCR reaction was ligated to pNN03, which had been previously linearized by EcoRV, dephosphorylated and fractionated through low temperature melting agarose. The plasmid pNN03 was constructed by removing the synthetic polylinker sequence from the commercially available plasmid pUC8 (Pharmacia, Piscataway, New Jersey) by restriction endonuclease cleavage and replacing the synthetic polylinker sequence with a novel synthetic sequence (SEQ ID NO:5).

The ligation mixture was used to transform E.coli K12 JA221(Iq) to ampicillin resistance. E.coli K12 JA221(Iq) cells are deposited with American Type Culture Collection (accession number 68845). Recombinant colonies were screened for the presence of an approximately 450 bp SacII fragment in the case of the heavy chain variable region, or an approximately 800 bp SacII fragment in the case of the light chain variable and constant regions. Some of the plasmids containing such fragments were sequenced.

The plasmid containing the sequence which corresponded to the amino terminal amino acid sequence of the 1E6 heavy chain variable region and which contained an in-frame variable region was designated plasmid pBAG188. The DNA and deduced amino acid sequences of the 1E6 heavy chain variable region are set forth in SEQ ID NO:3 and SEQ ID NO:4, respectively. The amino acid sequence was confirmed by direct protein sequencing data.

The plasmid containing the sequence which corresponded to the amino terminal amino acid sequence of the 1E6 kappa light chain and which contained in-frame kappa light chain variable and constant regions was designated plasmid pBAG169. The DNA and deduced amino acid sequences of the 1E6 light chain variable region are set forth in SEQ ID NO:1 and SEQ ID NO:2, respectively. This was confirmed by direct protein sequencing data.

Deposits

The following hybridoma cell lines were deposited under the Budapest Treaty with American Type Culture Collection (ATCC), Rockville, Maryland (USA) on March 5, 1991. The deposits are identified as follows:

| Culture | Antibody | Accession No. |
|---|---|---|
| 7A6-2E5 | 7A6 | HB 10695 |
| 1E6-2C12 | 1E6 | HB 10693 |
| HC-1B11-2E10 | HC-1B11 | HB 10694 |
| 8B8 | 8B8 | HB 10696 |

A bacteriophage and a bacterial strain carrying a vector, both encoding a DNA sequence referred to herein were deposited under the Budapest Treaty with In Vitro International, Inc., Linthicum, Maryland (USA) on May 28, 1987 and May 24, 1988, respectively, and assigned accession numbers IVI-10133 and IVI-10170, respectively. These deposits were transferred to the American Type Culture Collection on June 20, 1991, and are identified as follows:

| Designation | Accession No. | Description |
|---|---|---|
| λHT16[λgt10/LFA-3] | 75107 | Contain DNA encoding full length transmembrane LFA-3. |
| E.coli, BG8 | 68791 | |

A bacterial strain referred to herein was deposited under the Budapest Treaty with American Type Culture Collection on November 21, 1991 and identified as follows:

| Designation | Accession No. | Description |
|---|---|---|
| E.coli K12 JA221(lq) | 68845 | Bacterial host |

Sequences

The following is a summary of the sequences set forth in the Sequence Listing:

| SEQ ID NO:1 | DNA sequence of 1E6 light chain variable region |
|---|---|
| SEQ ID NO:2 | Amino acid sequence of 1E6 light chain variable region |
| SEQ ID NO:3 | DNA sequence of 1E6 heavy chain variable region |
| SEQ ID NO:4 | Amino acid sequence of 1E6 heavy chain variable region |
| SEQ ID NO:5 | DNA sequence of pNNO3 synthetic polylinker |
| SEQ ID NO:6 | DNA sequence of MKVP7 PCR primer |
| SEQ ID NO:7 | DNA sequence of 360-41 PCT primer |
| SEQ ID NO:8 | DNA sequence of VH01 PCR primer |
| SEQ ID NO:9 | DNA sequence of VH02 PCR primer |

While we have hereinbefore described a number of embodiments of this invention, it is apparent that our basic embodiments can be altered to provide other embodiments that utilize the compositions and processes of this invention. Therefore, it will be appreciated that the scope of this invention includes all alternative embodiments and variations which are defined in the foregoing specification and by the claims appended hereto; and the invention is not to be limited by the specific embodiments that have been presented herein by way of example.

## Claims

1. A monoclonal antibody produced by a hybridoma cell line formed by fusing cells of a mouse myeloma cell line with spleen cells from mice immunized with HT16/CHO cells expressing LFA-3, which antibody recognizes LFA-3 but does not recognize the same epitope as monoclonal antibody TS2/9.

2. The monoclonal antibody of claim 1 selected from antibodies produced by hybridomas:
   (a) 7A6-2E5 (ATCC HB 10695),
   (b) 8B8 (ATCC HB 10696),
   (c) HC-1B11-2E10 (ATCC HB 10694), and
   (d) 1E6-2C12 (ATCC HB 10693).

3. The monoclonal antibody of claim 1 or 2 wherein the mouse myeloma cell line is P3/X63-Ag8.653.

4. A monoclonal antibody having the characteristics of monoclonal antibody 7A6 (ATCC HB 10695) Fab, Fab', F(ab)$_2$, and F(v) fragments thereof, heavy chain monomers or dimers formed therefrom, light chain monomers or dimers formed therefrom, or dimers consisting of one heavy chain and one light chain formed therefrom, or humanized antibodies produced therefrom by CDR grafting.

18

EP 0 503 646 A1

**5.** A monoclonal antibody having the characteristics of monoclonal antibody HC-1B11 (ATCC HB 10694), Fab, Fab', F(ab)$_2$, and F(v) fragments thereof, heavy chain monomers or dimers formed therefrom, light chain monomers or dimers formed therefrom, or dimers consisting of one heavy chain and one light chain formed therefrom, or humanized antibodies produced therefrom by CDR grafting.

**6.** A monoclonal antibody having the characteristics of monoclonal antibody 8B8 (ATCC HB 10696), Fab, Fab', F(ab)$_2$, and F(v) fragments thereof, heavy chain monomers or dimers formed therefrom, light chain monomers or dimers formed therefrom, or dimers consisting of one heavy chain and one light chain formed therefrom, or humanized antibodies produced therefrom by CDR grafting.

**7.** A monoclonal antibody having the characteristics of monoclonal antibody 1E6 (ATCC HB 10693), Fab, Fab', F(ab)$_2$, and F(v) fragments thereof, heavy chain monomers or dimers formed therefrom, light chain monomers or dimers formed therefrom, or dimers consisting of one heavy chain and one light chain formed therefrom, or humanized antibodies produced therefrom by CDR grafting.

**8.** A monoclonal antibody that binds to LFA-3, binds to T11TS on sheep erythrocytes, and inhibits human T-lymphocyte binding to sheep erythrocytes.

**9.** A monoclonal antibody that binds to LFA-3, binds to T11TS, and inhibits binding of LFA-3 to CD2.

**10.** A hybridoma selected from the group consisting of
(a) 7A6-2E5 (ATCC HB 10695),
(b) 8B8 (ATCC HB 10696),
(c) HC-1B11-2E10 (ATCC HB 10694), and
(d) 1E6-2C12 (ATCC HB 10693).

**11.** A method for purifying LFA-3 comprising contacting a solution containing LFA-3 with a substrate on which is immobilized a monoclonal antibody according to any one of claims 1 to 9.

**12.** A method for separating cells expressing LFA-3 or T11TS comprising contacting a solution containing cells expressing LFA-3 or T11TS with a substrate on which is immobilized an antibody selected from 7A6, 8B8, or HC-1B11, Fab, Fab', F(ab)$_2$, or F(v) fragments thereof, heavy chain monomers or dimers formed therefrom, light chain monomers or dimers formed therefrom, or dimers consisting of one heavy chain and one light chain formed therefrom, or humanized antibodies produced therefrom by CDR grafting.

**13.** A method for detecting the presence, in a biological sample, of LFA-3 or cells expressing LFA-3 or homologues thereof, comprising contacting said biological sample with a detectably labeled antibody according to any one of claims 1 to 9.

**14.** A therapeutic reagent comprising a monoclonal antibody according to any one of claims 1 to 9.

**15.** A diagnostic kit comprising, as a reagent, a detectably labeled monoclonal antibody according to any one of claims 1 to 9.

**16.** A DNA sequence selected from the group consisting of: SEQ ID NO:1 (1E6 light chain variable region); SEQ ID NO:3 (1E6 heavy chain variable region); portions of either of the foregoing DNA sequences that encode a polypeptide that bind by themselves or with other polypeptides to LFA-3 at a different epitope than that recognized by monoclonal antibody TS2/9; and DNA sequences degenerate to any of the foregoing DNA sequences.

**17.** A recombinant DNA molecule comprising one or more of the DNA sequences according to claim 16 and one or more expression control sequences operatively linked thereto.

**18.** A host cell transformed with a recombinant DNA molecule according to claim 17.

**19.** A humanized recombinant antibody wherein the CDRs are as follows:
(1) the light chain CDR1 is AA$_{24}$-AA$_{34}$ of SEQ ID NO:2,

(2) the light chain CDR2 is $AA_{50}$-$AA_{56}$ of SEQ ID NO:2,

(3) the light chain CDR3 is $AA_{59}$-$AA_{97}$ of SEQ ID NO:2,

(4) the heavy chain CDR1 is $AA_{31}$-$AA_{35}$ of SEQ ID NO:4,

(5) the heavy chain CDR2 is $AA_{50}$-$AA_{56}$ of SEQ ID NO:4, and

(6) the heavy chain CDR3 is $AA_{99}$-$AA_{108}$ of SEQ ID NO:4; and

the humanized recombinant antibody is capable of binding to LFA-3.

20. A chimeric recombinant antibody wherein:

(a) the light chain variable region is SEQ ID NO:2; and

(b) the heavy chain variable region is SEQ ID NO:4.

21. A method of producing an anti-LFA-3 antibody comprising culturing a cell according to claim 10 or 18.

# Fig. 1

1A

1B

UNITS  FLUORESCENCE

# Fig. 1 (cont'd)

# Fig. 1 (cont'd)

Figure 2

Jurkat cells
+
SRBC

Jurkat cells
+
SRBC
+
nonspecific Ab

Jurkat cells
+
SRBC
+
TS2/9

Figure 2 (cont'd)

Jurkat cells
+
SRBC
+
1E6

Jurkat cells
+
SRBC
+
7A6

Jurkat cells
+
SRBC
+
8B8

Fig. 3

Fig. 4

# Fig. 5

# PARTIAL EUROPEAN SEARCH REPORT

which under Rule 45 of the European Patent Convention
shall be considered, for the purposes of subsequent
proceedings, as the European search report

Application number

## DOCUMENTS CONSIDERED TO BE RELEVANT

EP 92104318.8

| Category | Citation of document with indication, where appropriate, of relevant passages | Relevant to claim | CLASSIFICATION OF THE APPLICATION (Int Cl⁵) |
|---|---|---|---|
| X | EP - A - 0 280 578 (DANA FARBER CANCER INSTITUTE) * Claims 1-12 * -- | 1-13 | C 07 K 15/28 C 07 K 3/20 C 12 P 21/08 C 12 N 5/18 A 61 K 39/395 A 61 K 49/00 G 01 N 33/53 |
| A | CHEMICAL ABSTRACTS, vol. 111, no. 5, July 31, 1989, Columbus, Ohio, USA BIERER, BARBARA E. et al. "A monoclonal antibody to LFA-3, the CD2 ligand, specifically immobilizes major histocompatibility complex proteins." page 446, column 1, abstract-no. 37 543r & Eur.J.Immunol. 1989, 19(4), 661-5 -- | 1-21 | |
| A | CHEMICAL ABSTRACTS, vol. 108, no. 3, January 18, 1988, Columbus, Ohio, USA DENNING, STEPHEN M. et al. "Monoclonal antibodies to CD2 and lymphocyte function-asso- | 1-21 | |

TECHNICAL FIELDS SEARCHED (Int Cl⁵)

C 07 K 15/00
C 07 K 3/00
C 12 P 21/00
C 12 N 5/00
A 61 K 39/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12,14-21

Claims searched incompletely: 13 (Art. 52(4) EPC; diagnostic

Claims not searched: — method practised on the human

Reason for the limitation of the search: or animal body)

| Place of search | Date of completion of the search | Examiner |
|---|---|---|
| VIENNA | 29-05-1992 | SCHARF |

| DOCUMENTS CONSIDERED TO BE RELEVANT | | | CLASSIFICATION OF THE APPLICATION (Int. Cl.X) 5 |
|---|---|---|---|
| Category | Citation of document with indication, where appropriate, of relevant passages | Relevant to claim | |
| | ciated antigen 3 inhibit human thymic epithelial cell-dependent mature thymocyte activation." page 452, column 1, abstract-no. 20 236h    & J.Immunol. 1987, 139(8), 2573-8 -- | | |
| A | CHEMICAL ABSTRACTS, vol. 106, no. 11, March 16, 1987, Columbus, Ohio, USA VOLLGER, LEANNE WOLF et al. "Thymocyte binding to human thymic epithelial cells is inhibited by monoclonal antibodies to CD-2 and LFA-3 antigens." page 452, column 2, abstract-no. 82 796h    & J.Immunol. 1987, 138(2), 358-63 ---- | 1-21 | TECHNICAL FIELDS SEARCHED (Int. ClX) 5 |

SEQUENCE LISTING

(1) GENERAL INFORMATION:

    (i) APPLICANT: BIOGEN, INC.
                SATO, Vicki L.
                CHISHOLM, Patricia L.
                WALLNER, Barbara P.

    (ii) TITLE OF INVENTION: MONOCLONAL ANTIBODIES RECOGNIZING
          LYMPHOCYTE FUNCTION ASSOCIATED ANTIGEN-3

    (iii) NUMBER OF SEQUENCES: 9

    (iv) CORRESPONDENCE ADDRESS:
        (A) ADDRESSEE: c/o FISH & NEAVE
        (B) STREET: 875 Third Avenue
        (C) CITY: New York
        (D) STATE: New York
        (E) COUNTRY: U.S.A.
        (F) ZIP: 10022

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER:
        (B) FILING DATE:
        (C) CLASSIFICATION:

    (vii) PRIOR APPLICATION DATA:
        (A) APPLICATION NUMBER: US 07/667,975
        (B) FILING DATE: 12-MAR-1991

    (viii) ATTORNEY/AGENT INFORMATION:
        (A) NAME: HALEY Jr., James F.
        (B) REGISTRATION NUMBER: 27,794
        (C) REFERENCE/DOCKET NUMBER: B150CIP

    (ix) TELECOMMUNICATION INFORMATION:
        (A) TELEPHONE: (212) 715-0600
        (B) TELEFAX: (212) 715-0673
        (C) TELEX: 14-8367

(2) INFORMATION FOR SEQ ID NO:1:

    (i) SEQUENCE CHARACTERISTICS:
        (A) LENGTH: 327 base pairs
        (B) TYPE: nucleic acid
        (C) STRANDEDNESS: single
        (D) TOPOLOGY: linear

```
    (ix)  FEATURE:
          (A)  NAME/KEY: CDS
          (B)  LOCATION: 1..327

    (ix)  FEATURE:
          (A)  NAME/KEY: misc_feature
          (B)  LOCATION: 1..327
          (D)  OTHER INFORMATION: /note= "1E6 light chain variable
               region"


    (xi)  SEQUENCE DESCRIPTION: SEQ ID NO:1:

AAC ATT GTA ATG ACC CAA TCT CCC AAA TCC ATG TCC ATG TCA GTA GGA        48
Asn Ile Val Met Thr Gln Ser Pro Lys Ser Met Ser Met Ser Val Gly
 1               5                  10                  15

GAG AGG GTC ACC TTG ACC TGC AAG GCC AGT GAG AAT GTG GTT ACT TAT        96
Glu Arg Val Thr Leu Thr Cys Lys Ala Ser Glu Asn Val Val Thr Tyr
            20                  25                  30

GTT TCC TGG TAT CAA CAG AAA CCA GAG CAG TCT CCT AAA CTG CTG ATA       144
Val Ser Trp Tyr Gln Gln Lys Pro Glu Gln Ser Pro Lys Leu Leu Ile
            35                  40                  45

TAC GGG GCA TCC AAC CGG TAC ACT GGG GTC CCC GAT CGC TTC ACA GGC       192
Tyr Gly Ala Ser Asn Arg Tyr Thr Gly Val Pro Asp Arg Phe Thr Gly
        50                  55                  60

AGT GGA TCT GCA ACA GAT TTC ACT CTG ACC ATC AGC AGT GTG CAG GCT       240
Ser Gly Ser Ala Thr Asp Phe Thr Leu Thr Ile Ser Ser Val Gln Ala
 65                 70                  75                  80

GAA GAC CTT GCA GAT TAT CAC TGT GGA CAG GGT TAC AGC TAT CCG TAC       288
Glu Asp Leu Ala Asp Tyr His Cys Gly Gln Gly Tyr Ser Tyr Pro Tyr
                85                  90                  95

ACG TTC GGA GGG GGG ACC AAG CTG GAA ATA AAA CGG GCT                   327
Thr Phe Gly Gly Gly Thr Lys Leu Glu Ile Lys Arg Ala
            100                 105



(2) INFORMATION FOR SEQ ID NO:2:

          (i)  SEQUENCE CHARACTERISTICS:
               (A)  LENGTH: 109 amino acids
               (B)  TYPE: amino acid
               (D)  TOPOLOGY: linear

          (ii)  MOLECULE TYPE: protein

          (xi)  SEQUENCE DESCRIPTION: SEQ ID NO:2:

Asn Ile Val Met Thr Gln Ser Pro Lys Ser Met Ser Met Ser Val Gly
 1               5                  10                  15
```

```
Glu Arg Val Thr Leu Thr Cys Lys Ala Ser Glu Asn Val Val Thr Tyr
            20                  25                  30

Val Ser Trp Tyr Gln Gln Lys Pro Glu Gln Ser Pro Lys Leu Leu Ile
            35                  40                  45

Tyr Gly Ala Ser Asn Arg Tyr Thr Gly Val Pro Asp Arg Phe Thr Gly
        50                  55                  60

Ser Gly Ser Ala Thr Asp Phe Thr Leu Thr Ile Ser Ser Val Gln Ala
65                  70                  75                  80

Glu Asp Leu Ala Asp Tyr His Cys Gly Gln Gly Tyr Ser Tyr Pro Tyr
                85                  90                  95

Thr Phe Gly Gly Gly Thr Lys Leu Glu Ile Lys Arg Ala
                100                 105
```

(2) INFORMATION FOR SEQ ID NO:3:

    (i) SEQUENCE CHARACTERISTICS:
        (A) LENGTH: 357 base pairs
        (B) TYPE: nucleic acid
        (C) STRANDEDNESS: single
        (D) TOPOLOGY: linear

    (ix) FEATURE:
        (A) NAME/KEY: CDS
        (B) LOCATION: 1..357

    (ix) FEATURE:
        (A) NAME/KEY: misc_feature
        (B) LOCATION: 1..357
        (D) OTHER INFORMATION: /note= "1E6 heavy chain variable
            region"

    (xi) SEQUENCE DESCRIPTION: SEQ ID NO:3:

```
NNN GTG AAA CTG CAG GAG TCA GGG CCT GAG CTG GTG AGG CCT GGG GAA        48
Xaa Val Lys Leu Gln Glu Ser Gly Pro Glu Leu Val Arg Pro Gly Glu
 1               5                   10                  15

TCA GTG AAG ATT TCC TGC AAG GGT TCC GGC TAC ACA TTC ACT GAT TAT        96
Ser Val Lys Ile Ser Cys Lys Gly Ser Gly Tyr Thr Phe Thr Asp Tyr
            20                  25                  30

GCT ATA CAC TGG GTG AAG CAG AGT CAT GCA AAG AGT CTA GAG TGG ATT       144
Ala Ile His Trp Val Lys Gln Ser His Ala Lys Ser Leu Glu Trp Ile
            35                  40                  45

GGA GTT ATT AGT GTT CAC TAT GAT AAA ACA AAC TAC AAC CAG AAG TTT       192
Gly Val Ile Ser Val His Tyr Asp Lys Thr Asn Tyr Asn Gln Lys Phe
        50                  55                  60
```

```
AAG GGC AAG GCC TCA ATG ACT GTA GAC AAA TCC TCC AGC ACA GCC TAT      240
Lys Gly Lys Ala Ser Met Thr Val Asp Lys Ser Ser Ser Thr Ala Tyr
 65              70                  75                  80

ATG GAA CTT GCC AGA TTG ACA TCT GAG GAT TCT GCC ATC TAT TAC TGT      288
Met Glu Leu Ala Arg Leu Thr Ser Glu Asp Ser Ala Ile Tyr Tyr Cys
                 85                  90                  95

GCA AGA TCC TTT TAC TAC GGT AGG GAC TTT GAC AAC TGG GGC CAA GGG      336
Ala Arg Ser Phe Tyr Tyr Gly Arg Asp Phe Asp Asn Trp Gly Gln Gly
             100                 105                 110

ACC ACG GTC ACC GTC TCC TCA                                          357
Thr Thr Val Thr Val Ser Ser
         115
```

(2) INFORMATION FOR SEQ ID NO:4:

    (i) SEQUENCE CHARACTERISTICS:
        (A) LENGTH: 119 amino acids
        (B) TYPE: amino acid
        (D) TOPOLOGY: linear

    (ii) MOLECULE TYPE: protein

    (xi) SEQUENCE DESCRIPTION: SEQ ID NO:4:

```
Xaa Val Lys Leu Gln Glu Ser Gly Pro Glu Leu Val Arg Pro Gly Glu
 1               5                  10                  15

Ser Val Lys Ile Ser Cys Lys Gly Ser Gly Tyr Thr Phe Thr Asp Tyr
                 20                  25                  30

Ala Ile His Trp Val Lys Gln Ser His Ala Lys Ser Leu Glu Trp Ile
             35                  40                  45

Gly Val Ile Ser Val His Tyr Asp Lys Thr Asn Tyr Asn Gln Lys Phe
         50                  55                  60

Lys Gly Lys Ala Ser Met Thr Val Asp Lys Ser Ser Ser Thr Ala Tyr
 65              70                  75                  80

Met Glu Leu Ala Arg Leu Thr Ser Glu Asp Ser Ala Ile Tyr Tyr Cys
                 85                  90                  95

Ala Arg Ser Phe Tyr Tyr Gly Arg Asp Phe Asp Asn Trp Gly Gln Gly
             100                 105                 110

Thr Thr Val Thr Val Ser Ser
         115
```

16

(2) INFORMATION FOR SEQ ID NO:5:

    (i) SEQUENCE CHARACTERISTICS:
        (A) LENGTH: 115 base pairs
        (B) TYPE: nucleic acid
        (C) STRANDEDNESS: single
        (D) TOPOLOGY: linear

    (ix) FEATURE:
        (A) NAME/KEY: misc_feature
        (B) LOCATION: 1..115
        (D) OTHER INFORMATION: /note= "pNN03 synthetic linker"

    (xi) SEQUENCE DESCRIPTION: SEQ ID NO:5:

GCGGCCGCGG TCCAACCACC AATCTCAAAG CTTGGTACCC GGGAATTCAG ATCTGCAGCA        60

TGCTCGAGCT CTAGATATCG ATTCCATGGA TCCTCACATC CCAATCCGCG GCCGC        115


(2) INFORMATION FOR SEQ ID NO:6:

    (i) SEQUENCE CHARACTERISTICS:
        (A) LENGTH: 41 base pairs
        (B) TYPE: nucleic acid
        (C) STRANDEDNESS: single
        (D) TOPOLOGY: linear

    (ix) FEATURE:
        (A) NAME/KEY: misc_feature
        (B) LOCATION: 1..41
        (D) OTHER INFORMATION: /note= "MKVP7 PCR primer"

    (xi) SEQUENCE DESCRIPTION: SEQ ID NO:6:

ACTAGTCGAC ATGGGCWTCA AGATGGAGTC ACAKWYYCWG G        41


(2) INFORMATION FOR SEQ ID NO:7:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

    (ix) FEATURE:
        (A) NAME/KEY: misc_feature
        (B) LOCATION: 1..34
        (D) OTHER INFORMATION: /note= "360-41 PCR primer"

    (xi) SEQUENCE DESCRIPTION: SEQ ID NO:7:

GCGCCGTCTA GAATTAACAC TCATTCCTGT TGAA        34

```
(2) INFORMATION FOR SEQ ID NO:8:

        (i) SEQUENCE CHARACTERISTICS:
            (A) LENGTH: 22 base pairs
            (B) TYPE: nucleic acid
            (C) STRANDEDNESS: single
            (D) TOPOLOGY: linear

        (ix) FEATURE:
            (A) NAME/KEY: misc_feature
            (B) LOCATION: 1..22
            (D) OTHER INFORMATION: /note= "VH01 PCR primer"

        (xi) SEQUENCE DESCRIPTION: SEQ ID NO:8:

    AGGTSMARCT GCAGSAGTCW GG                                        22



(2) INFORMATION FOR SEQ ID NO:9:

        (i) SEQUENCE CHARACTERISTICS:
            (A) LENGTH: 32 base pairs
            (B) TYPE: nucleic acid
            (C) STRANDEDNESS: single
            (D) TOPOLOGY: linear

        (ix) FEATURE:
            (A) NAME/KEY: misc_feature
            (B) LOCATION: 1..32
            (D) OTHER INFORMATION: /note= "VH02 PCR primer"

        (xi) SEQUENCE DESCRIPTION: SEQ ID NO:9:

    TGAGGAGACG GTGACCGTGG TCCCTTGGCC CC                             32
```